# EUROPEAN PATENT APPLICATION

(11) **EP 4 800 114 A1**
(43) Date of publication of application: **02.09.2026**
(21) Application number: 24881618.3
(22) Date of filing: 23.10.2024
(51) Int. Cl.: C12N 15/85, A61K 48/00, A61K 39/00, A61K 31/7105

(54) **PLASMID VECTOR FOR IN-VITRO TRANSCRIPTION OF MRNA, CONSTRUCTION METHOD THEREFOR AND USE THEREOF**

(30) Priority: 23.10.2023 CN 202311375951
(71) Applicant: Cansino (Shanghai) Biological Research Co., Ltd., Shanghai 201203 (CN)
(72) Inventor: LI, Jin, Shanghai 201203 (CN); LIU, Xingpo, Shanghai 201203 (CN); WU, Han, Shanghai 201203 (CN); WANG, Haomeng, Shanghai 201203 (CN); LIU, Jian, Shanghai 201203 (CN); ZHU, Tao, Shanghai 201203 (CN); QIU, Dongxu, Shanghai 201203 (CN); YU, Xuefeng, Shanghai 201203 (CN)
(74) Representative: Finnegan Europe LLP
(86) International application number: PCT/CN2024/126613
(87) International publication number: WO 2025/087253

(57) **Abstract**

Provided is a nucleic acid molecule, comprising 1) a transcribable nucleic acid fragment and/or a nucleic acid used for introducing a transcribable nucleic acid fragment; 2) a polyA nucleic acid fragment; and 3) a gyrase binding site nucleic acid fragment. The nucleic acid molecule can improve the stability of polyA, thus solving the problem of polyA loss/shortening. Additionally, the present invention improves the stability of nucleic acid molecules, thus reducing the probability of recombination of plasmids during amplification, and facilitating industrial production of mRNA products.

## Description

### TECHNICAL FIELD

The present disclosure belongs to the field of genetic engineering. Specifically, the present disclosure relates to an *in vitro* mRNA transcription vector capable of improving plasmid stability, a construction method, and use thereof.

### BACKGROUND

The mRNA technology offers many advantages: firstly, mRNA will not be integrated into the genome, thereby avoiding concerns about insertion mutations; more importantly, unlike relatively stable DNA, mRNA degrades after functioning, causing no other toxic or side effects to the human body; mRNA can be produced in a cell-free manner, and can be rapidly and economically produced on a large scale by the *in vitro* transcription technology. Its short research and development cycle enables rapid, economic, and efficient production. In addition, a single mRNA can encode multiple antigens, proteins, or polypeptides, and the process has high applicability. Theoretically, any protein can be expressed.
mRNA vaccines against COVID-19 have verified the applicability of the mRNA technology platform in the vaccine field. In addition to vaccines against COVID-19, mRNA technology can also be widely applied to the fields of infectious diseases, tumors, protein replacement therapy, and the like.

For mRNA technology, a good production process starts with the design of safe, stable, and efficient plasmid vectors. Most of the unnecessary sequences in the plasmid DNA should be removed to reduce the molecular weight as much as possible for genetic manipulation, whether from the perspective of the process production or from the perspective of treatment or regulation. This is because an overly large plasmid will impose a huge metabolic load on the host bacterium and reduce the resources used by the cells for plasmid replication, resulting in a decrease in the plasmid DNA yield. In addition, in industrial production, the plasmid must maintain a high degree of stability during the continuous passage process to ensure the wide application and stability of mRNA technology.

There is a challenge in the production process using mRNA plasmid technology: the PolyA sequence of the plasmid is shortened with the continuous amplification of bacteria, which will cause trouble to the cloning and amplification of plasmids carrying the PolyA tail. This makes it impossible to achieve the uniformity of the PolyA sequences of the produced plasmids and fails to meet the GMP-level production process and drug regulatory requirements. Moreover, the PolyA sequence is also a key element for mRNA stability and translation and expression efficiency. Therefore, solving the problem of PolyA sequence shortening in plasmid fermentation production has been a persistent goal that researchers have been striving to achieve.

To eliminate the instability of the PolyA tail on the circular plasmid, Kyle Jacoby et al. developed the pEVL linear plasmid system derived from the PJazz plasmid. The pEVL plasmid has a significant advantage in the maintenance of the PolyA tail length compared to the traditional circular plasmid upon continuous subculture (Kyle Jacoby, 2016, Mol Ther Nucleic Acids). However, the pEVL plasmid has a very low copy number and is not suitable for large-scale production. In 2019, Zeljka Trepotec et al. found that interspersing the PolyA sequence with spacers can reduce recombination that occurs during plasmid DNA amplification, maintaining the length of the PolyA tail. The use of a segmented polyA tail is exemplified by the technical solution disclosed in Patent US10717982B2 of BioNTech and the technique disclosed in Patent WO2020074642A1 of Ethris. Kristie Bloom et al. developed a plasmid capable of increasing the length of PolyA by enzyme digestion and ligation. The plasmid was used as a template for *in vitro* co-transcription of mRNA to ameliorate the situation where the length of PolyA is shortened during plasmid passage. In addition, some other companies have used a relatively short polyA sequence to stabilize plasmids.

However, most of the methods described above are improvements to polyA itself, and even the plasmid system developed to ameliorate the instability of PolyA has proved unsuitable for large-scale production and use.

### SUMMARY

The present disclosure carries out further research on plasmids suitable for RNA technology, moving beyond most existing improvement methods that focus only on polyA. By considering the plasmid as an integrated entity, the present disclosure solves the problem of PolyA loss without affecting plasmid replication and yield, thus improving plasmid integrity and stability and rendering it suitable for large-scale production.

The present disclosure provides a nucleic acid molecule for improving the stability of a PolyA tail during plasmid passage in bacterial fermentation and use thereof.

The term "vector" described herein has the general meaning known to those skilled in the art herein and includes any intermediate vector of a nucleic acid, for example, a vector that enables the nucleic acid to be introduced into a prokaryotic and/or eukaryotic host cell or to be integrated into a genome where appropriate, and the vector is preferably replicated and/or expressed in the cell.

The term "pharmaceutically acceptable carrier" described herein refers to an inactive substance formulated with a pharmaceutically active substance to meet or facilitate the fulfillment of the requirements of the active substance in terms of dose, adsorption, solubility, or pharmacokinetics.

The term "plasmid" described herein generally relates to a construct of extrachromosomal genetic material, typically a circular DNA duplex, which can replicate independently of chromosomal DNA.

The term "host cell" described herein refers to any cell that can be transformed or transfected with an exogenous nucleic acid, including prokaryotic cells (e.g., *E*. *coli*) or eukaryotic cells (e.g., yeast cells and insect cells). Particularly preferred are mammalian cells, e.g., cells from humans, mice, hamsters, pigs, goats, or primates.

Cells may be derived from a variety of tissue types and include primary cells and cell lines.

The terms "peptide" and "protein" described herein include substances containing not only amino acid components but also non-amino acid components such as sugar and phosphate structures, and also include substances containing bonds such as ester bonds, thioether bonds, or disulfide bonds.

The term "nucleotide fragment encoding a peptide or a protein" described herein refers to a nucleotide fragment which, when present in a suitable environment, preferably in a cell, can direct the assembly of amino acids during translation to produce the peptide or the protein.

The term "immune response" described herein relates to the reaction of the immune system to, for example, an immunogenic organism such as bacteria or viruses, cells, or substances. The term "immune response" includes innate immune responses and adaptive immune responses. Preferably, the immune response is associated with the activation of immune cells, the induction of cytokine biosynthesis, and/or antibody production. Preferably, the immune response comprises the steps of activating antigen presenting cells such as dendritic cells and/or macrophages, presenting an antigen or a fragment thereof by the antigen presenting cells, and activating cytotoxic T cells as a result of the presentation.

In a first aspect of the present disclosure, provided is a nucleic acid molecule, which includes: 1) a transcribable nucleic acid fragment or a nucleic acid for introducing a transcribable nucleic acid fragment; 2) a nucleic acid fragment comprising polydeoxyadenosine polyA; and 3) a nucleic acid fragment comprising a gyrase binding site (SGS).

The transcribable nucleic acid fragment includes a nucleic acid fragment encoding a peptide or a protein.

The nucleic acid for introducing the transcribable nucleic acid fragment comprises a multiple cloning site or a homologous recombination site.

The polyA comprises at least 50 contiguous deoxyadenosines.

The nucleic acid fragment comprising the polyA comprises at least two elements having a length of at least 20 contiguous deoxyadenosine sequences, and the two elements are spaced apart by at least one non-T deoxynucleotide.

In some specific embodiments, the nucleic acid fragment comprising the gyrase binding site is derived from a virus or a bacterium; preferably, the nucleic acid fragment comprising the gyrase binding site is derived from a virus; more preferably, the nucleic acid fragment comprising the gyrase binding site is derived from a phage. In some specific embodiments, the nucleic acid fragment comprising the gyrase binding site is derived from a plasmid; preferably, the nucleic acid fragment comprising the gyrase binding site is derived from a ColE1 plasmid.

In some specific embodiments, the SGS nucleic acid fragment is constructed upstream of a T7 promoter in a plasmid vector; in some specific embodiments, the SGS nucleic acid fragment is constructed downstream of a PolyA in a plasmid vector. Those skilled in the art can select an appropriate position to insert the SGS according to the present disclosure.

In another aspect, the present disclosure provides a DNA expression vector or a recombinant plasmid, wherein the sequence of the DNA expression vector or the recombinant plasmid comprises: 1) a first nucleic acid fragment encoding an mRNA molecule; 2) a second nucleic acid fragment comprising a polydeoxyadenosine polyA; and 3) a third nucleic acid fragment comprising a gyrase binding site.

The present disclosure further provides a method for preparing an expression vector or a recombinant plasmid, which comprises: transforming the expression vector or the recombinant plasmid described above into *E*. *coli* competent cells, spreading a proper amount of the transformed cells onto a resistant LB culture medium plate for culturing, picking a monoclonal colony and performing sequencing to obtain a positive bacterial solution verified to be correct by sequencing, and then conducting large-scale culturing.

The present disclosure provides a method for amplifying a nucleic acid molecule, which comprises replicating the nucleic acid molecule described above in a host cell and propagating the host cell.

In some specific embodiments, the host cell is a eukaryotic cell or a prokaryotic cell; preferably, the host cell is *E*. *coli*.

The present disclosure provides a method for obtaining an RNA, which comprises amplifying a nucleic acid molecule by the method described above, and transcribing the nucleic acid molecule *in vitro* as a template into the RNA.

The present disclosure provides a method for obtaining a peptide or a protein, which comprises obtaining an mRNA encoding the peptide or the protein by the method for obtaining an RNA described above, and translating the mRNA into an amino acid fragment of the peptide or the protein.

The present disclosure provides a system for producing a eukaryotic translatable mRNA, which comprises: 1) a vector or plasmid comprising the nucleic acid molecule described above; and 2) a host cell.

In another aspect, the present disclosure provides use of a nucleic acid nucleotide fragment encoding a gyrase binding site in constructing a plasmid or a vector, wherein the plasmid or the vector is used for *in vitro* transcription into RNA.

In another aspect, the present disclosure provides a composition, which comprises an RNA, a peptide, or a protein obtained by the method described above.

In some specific embodiments, the composition comprises one or more of a pharmaceutically acceptable excipient, a carrier, a buffer, a protectant, a stabilizer, a surfactant, an osmotic pressure regulator, an adjuvant, a preservative, and an inactivator.

The buffer described herein includes one or more of HEPES, HIS, TRIS, PB, succinic acid, and citric acid; the protectant includes one or more of gelatin, ethanol, ethylene diamine tetraacetic acid (EDTA), disodium ethylene diamine tetraacetic acid (EDTA-2Na), and magnesium chloride; the stabilizer includes one or more of sucrose, mannitol, fucose, and maltose; the surfactant includes one or more of Tween, Span, and glycerol; the osmotic pressure regulator includes sodium chloride or is omitted; the excipient component includes one or more of mannitol, sucrose, sodium chloride, magnesium chloride, HEPES, polysorbate 80, and glycerol.

In some specific embodiments, the composition described herein may be prepared as a mucosal immune formulation, a humoral immune formulation, a cellular immune formulation, or a cutaneous immune formulation.

In some specific embodiments, the composition described herein may be prepared in a liquid dosage form, a solid dosage form, a semi-solid dosage form, a gaseous dosage form, or an inhalation dosage form.

In some specific embodiments, the composition described herein is administered by intravenous injection, intramuscular injection, subcutaneous injection, oral administration, buccal administration, sublingual administration, rectal administration, respiratory administration, or transcutaneous administration; the respiratory administration is oral inhalation, nasal inhalation, or inhalation after atomization by an atomization and administration device.

The nucleic acid molecule and the plasmid or the vector comprising the nucleic acid molecule of the present disclosure can be used for the expression of a recombinant protein in cellular transcription and expression. More specifically, when a recombinant protein is produced, an expression vector of the present disclosure may be used to transcribe a recombinant nucleic acid and express the recombinant protein in a cell-based system, for example, the preparation of recombinant antibodies, hormones, cytokines, enzymes, and the like.

The nucleic acid molecule and the plasmid or the vector comprising the nucleic acid molecule of the present disclosure can be used, for example, for transient expression of genes, and a possible application field is RNA-based vaccines, wherein the nucleic acid molecule or plasmid/vector is transfected into cells *in vitro* or administered directly *in vivo* or *in vitro* to transiently express functional proteins.

The present disclosure provides uses of the composition described above, including use in medicine, in delivering a transgene into a host cell, in eliciting a primary immune response in an animal to treat or prevent at least one disease, in potentiating an immune response in an animal, and in inducing an immune response in an animal that breaks tolerance to self-antigens. The immune response includes vaccines for preventing cancer, therapeutic tumor vaccines, and vaccines for preventing viral infection, bacterial infection, and fungal infection.

The present disclosure provides use of any one of the nucleic acid molecules, expression vectors, or compositions described above in preparing a medicament or a vaccine for treating or preventing a disease.

In some specific embodiments, the disease is caused by one or more of the following viruses, bacteria, or protozoa: novel coronavirus, dengue virus, malaria parasite, herpes zoster virus, feline parvovirus, feline calicivirus, feline herpes virus, feline leukemia virus, feline coronavirus, porcine reproductive and respiratory syndrome virus, herpes simplex virus 1, herpes simplex virus 2, encephalitis virus, papillomavirus, varicella zoster virus, Epstein-Barr virus, human cytomegalovirus, human herpes virus 8, human papillomavirus, BK virus, JC virus, smallpox, poliovirus, hepatitis B virus, human bocavirus, parvovirus B19, human astrovirus, Norwalk virus, coxsackievirus, hepatitis A virus, rhinovirus, severe acute respiratory syndrome virus, hepatitis C virus, yellow fever virus, dengue virus, West Nile virus, rubella virus, hepatitis E virus, human immunodeficiency virus (HIV), influenza virus A or B, Guanarito virus, Junin virus, Lassa virus, Machupo virus, Sabia virus, Crimean-Congo hemorrhagic fever virus, Ebola virus, Marburg virus, measles virus, mumps virus, parainfluenza virus, respiratory syncytial virus, human metapneumovirus, Hendra virus, Nipah virus, rabies virus, hepatitis D, rotavirus, orbivirus, *E*. *coli* virus, human enterovirus, Japanese encephalitis virus, vesicular herpes virus, eastern equine encephalitis virus, Hantaan virus, Middle East respiratory syndrome coronavirus, chikungunya virus, or Banna virus.

The present disclosure has the following beneficial effects.

In a first aspect, the stability of the PolyA is improved by the SGS sequence, so as to solve the problem of PolyA loss/shortening.

In a second aspect, the stability of the nucleic acid molecule is improved by the SGS sequence.

In a third aspect, the SGS sequence reduces the probability of the recombination of a plasmid during amplification, further improving the safety of plasmid application. In a fourth aspect, through the SGS sequence, an RNA molecule, a peptide, or a protein that is of higher purity and meets GMP-level production processes and drug regulatory requirements can be obtained, which is suitable for large-scale production.

### BRIEF DESCRIPTION OF THE DRAWINGS

FIG. 1 shows the effect of SGS sequences from different sources on the stability of polyA sequence under antibiotic-free conditions.
FIG. 2 shows the effect of SGS sequences from different sources on the stability of polyA sequence under kanamycin resistance conditions.
FIG. 3 shows the effect of the SGS Mu398 sequence on the stability of PolyA of different lengths under antibiotic-free conditions.
FIG. 4 shows the effect of the SGS Mu398 sequence on the stability of PolyA of different lengths under kanamycin resistance conditions.
FIG. 5 shows the applicability of the SGS Mu398 sequence to antigen sequences for different purposes under antibiotic-free conditions.
FIG. 6 shows the applicability of the Mu398 SGS sequence to antigen sequences for different purposes under kanamycin resistance conditions.
FIG. 7 shows the detection results of the mRNA integrity after transcription from the 10^{th} generation of the F001 recombinant plasmid.
FIG. 8 shows the detection results of the mRNA integrity after transcription from the 10^{th} generation of the F002 recombinant plasmid.
FIG. 9 shows the detection results of the mRNA integrity after transcription from the 10^{th} generation of the F003 recombinant plasmid.
FIG. 10 shows the detection results of the mRNA integrity after transcription from the 10^{th} generation of the F004 recombinant plasmid.
FIG. 11 shows the detection results of the mRNA integrity after transcription from the 10^{th} generation of the F005 recombinant plasmid.

### DETAILED DESCRIPTION

### Example 1. Effect of SGS Sequences from Different Sources on Stability of Plasmid polyA

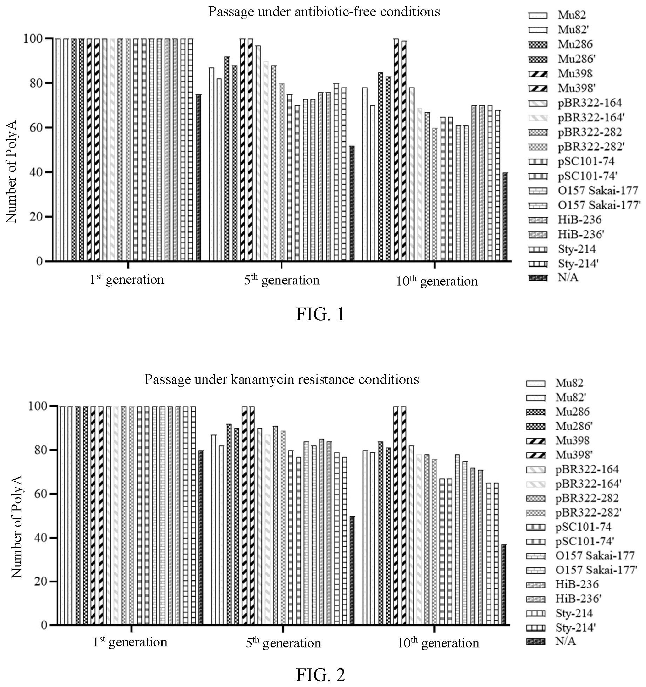

To investigate the effect of the SGS sequence on plasmid stability, the SGS sequences from different sources were each inserted downstream of a vector polyA or upstream of a T7 promoter, wherein the polyA was a continuous 100A sequence, and meanwhile, a target gene sequence was inserted into the plasmid vector. The effect of the SGS sequence on plasmid stability was observed. The design is shown in Table 1. The results show that the insertion of the SGS sequence was capable of significantly improving the passage stability of polyA under both antibiotic-free conditions and kanamycin resistance conditions (FIGs. 1 and 2).

The transcription vector of the present disclosure comprised a 5' UTR sequence, a Kozak sequence, an antigen-encoding sequence, a 3' UTR sequence, a polyA tail sequence, and an SGS sequence, wherein the sequences were individually synthesized into a pUC57-kana vector by Nanjing GenScript Biotech Co., Ltd., and then sequentially linked to a universal transcription vector by homologous recombination. The constructed plasmid was transformed into Trans1-Blue *E*. *coli* competent cells. A proper amount of the transformed cells were spread onto a Kanaresistant LB culture medium plate and cultured at 37 °C for a certain period of time. A monoclonal colony was picked and sequenced, and the positive bacterial solution verified to be correct by sequencing was stored. The bacterial solution containing the positive plasmid was then shaken at 37 °C, large-scale plasmid extraction was performed to obtain an *in vitro* transcription template, and mRNA was prepared by co-transcriptional capping.

**Table 1. Design for investigating the effect of SGS sequence on plasmid stability**

| No. | PolyA | Gyrase binding site | Insertion position | Sequence |
|---|---|---|---|---|
| 1 | | Mu82 | Downstream of PolyA | SEQ ID NO: 1 |
| 2 | | Mu82' | Upstream of T7 promoter | |
| 3 | | Mu286 | Downstream of PolyA | SEQ ID NO: 2 |
| 4 | | Mu286' | Upstream of T7 promoter | |
| 5 | | Mu398 | Downstream of PolyA | SEQ ID NO: 3 |
| 6 | | Mu398' | Upstream of T7 promoter | |
| 7 | | pBR322-164 | Downstream of PolyA | SEQ ID NO: 4 |
| 8 | | pBR322-164' | Upstream of T7 promoter | |
| 9 | | pBR322-282 | Downstream of PolyA | SEQ ID NO: 5 |
| 10 | 100A | pBR322-282' | Upstream of T7 promoter | |
| 11 | | pSC101-74 | Downstream of PolyA | SEQ ID NO: 6 |
| 12 | | pSC101-74' | Upstream of T7 promoter | |
| 13 | | O157 Sakai-177 | Downstream of PolyA | SEQ ID NO: 7 |
| 14 | | O157 Sakai-177' | Upstream of T7 promoter | |
| 15 | | HiB-236 | Downstream of PolyA | SEQ ID NO: 8 |
| 16 | | HiB-236' | Upstream of T7 promoter | |
| 17 | | Sty-214 | Downstream of PolyA | SEQ ID NO: 9 |
| 18 | | Sty-214' | Upstream of T7 promoter | |
| Control group | | None | N/A | N/A |

### Example 2. Effect of SGS Sequence on Stability of PolyA of Different Lengths

To further verify whether the SGS sequence was able to improve the stability of PolyA sequences of different lengths, the Mu398 SGS sequence and different types of polyA sequences were inserted into the plasmid vector described above, and the passage stability of the polyA sequences was observed. The specific design is shown in Table 2.

**Table 2. Design for investigating the effect of SGS sequence on stability of PolyA of different lengths**

| Group | No. | Gyrase binding site (SGS) | PolyA |
|---|---|---|---|
| Experimental group | 1 | Mu398 | 60A |
| | 2 | | 80A |
| | 3 | | 100A |
| | 4 | | 120A |
| | 5 | | 150A |
| | 6 | | 30A*2 |
| | 7 | | 30A*3 |
| | 8 | | 40A*2 |
| | 9 | | 40A*3 |
| | 10 | | 50A*2 |
| | 11 | | 50A*3 |
| | 12 | | 60A*2 |
| Control group | 1 | None | 60A |
| | 2 | | 80A |
| | 3 | | 100A |
| | 4 | | 120A |
| | 5 | | 150A |
| | 6 | | 30A*2 |
| | 7 | | 30A*3 |
| | 8 | | 40A*2 |
| | 9 | | 40A*3 |
| | 10 | | 50A*2 |
| | 11 | | 50A*3 |
| | 12 | | 60A*2 |

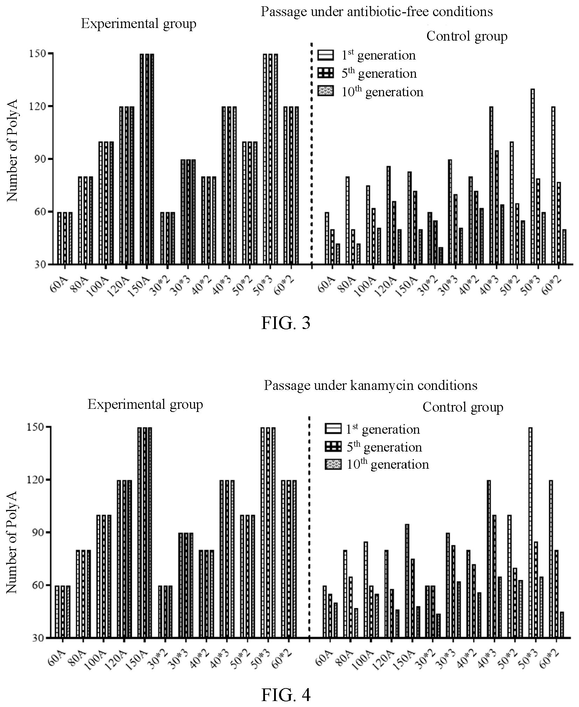

The results show that the addition of the Mu398 SGS sequence was able to increase the passage stability of various types of polyA sequences and maintain their integrity within 10 generations (FIGs. 3 and 4).

### Example 3. Determination of Universal Plasmid Vector with High Stability

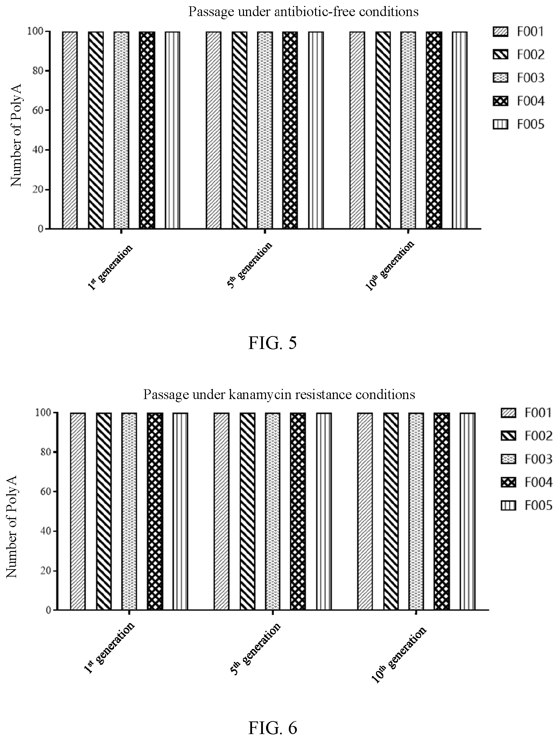

To further verify the universality of the plasmid vector described above, target gene sequences from 5 different pathogen sources, namely novel coronavirus (COVID-19), dengue virus (DENV), malaria, varicella zoster virus (VZV), and feline parvovirus (FPV), were separately inserted into the vector described above containing the SGS sequence to construct expression vectors F001, F002, F003, F004, and F005, respectively. The passage stability of continuous 100A during plasmid passage was observed. The results show that the SGS sequence was able to be commonly used for different target gene sequences and was capable of ensuring the passage stability of each recombinant plasmid polyA within 10 generations (as shown in FIGs. 5 and 6).

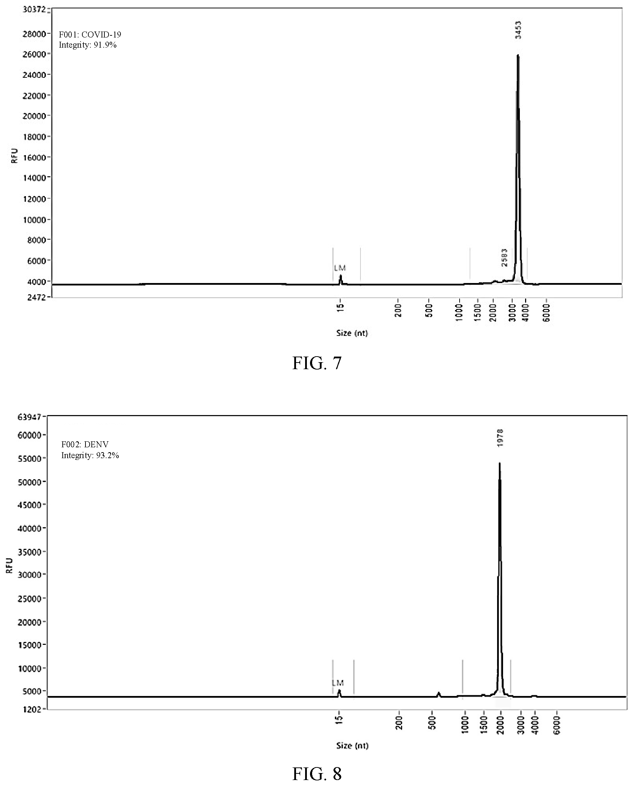

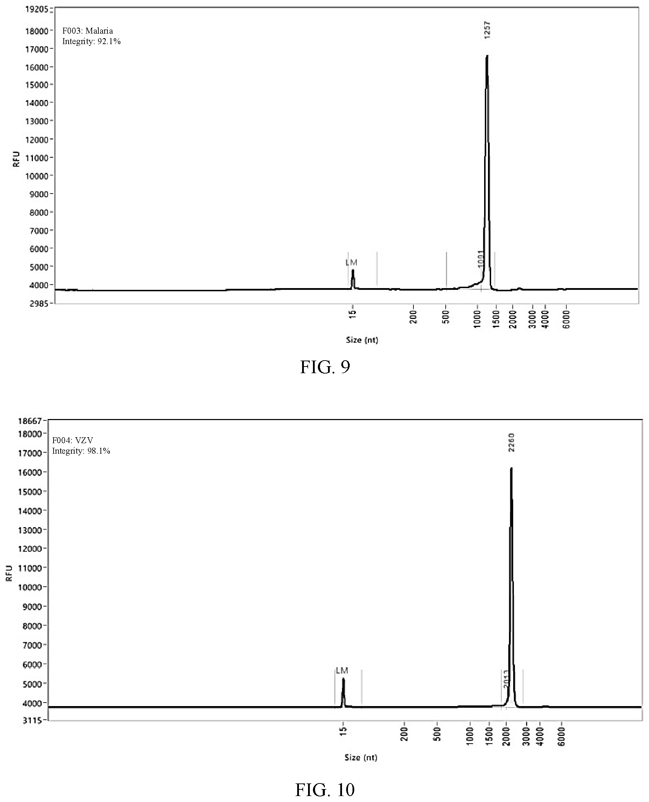

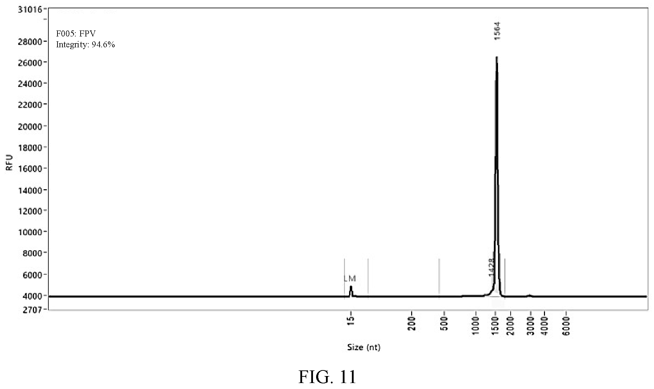

To further observe the transcription effect of the transcription vector, the 10^{th} generation of the recombinant plasmid constructed above was subjected to large-scale extraction and then used for *in vitro* transcription. The results show that each plasmid was able to transcribe high-quality mRNA (as shown in FIGs. 7-11, see Table 3 for data).

**Table 3. Data for detection results in FIGs. 7-11**

| Peak | Size (nt) | From (nt) | To (nt) | Relative concentration percentage | Concentration (ng/µL) | Molar concentration (nmole/mL) |
|---|---|---|---|---|---|---|
| FIG. 7 | | | | | | |
| 1 | 15 (LM) | 0 | 79 | | 0.5194 | 104.5892 |
| 2 | 2583 | 1290 | 3001 | 8.1 | 16.3378 | 21.5772 |
| 3 | 3453 | 3001 | 4185 | 91.9 | 184.9824 | 167.7085 |

| FIG. 8 | | | | | | |
|---|---|---|---|---|---|---|
| 1 | 15 (LM) | 0 | 61 | | 0.2057 | 36.6907 |
| 2 | 1858 | 977 | 1866 | 6.8 | 5.2221 | 10.6669 |
| 3 | 1978 | 1866 | 2513 | 93.2 | 72.0742 | 113.3759 |

| FIG. 9 | | | | | | |
|---|---|---|---|---|---|---|
| 1 | 15 (LM) | 1 | 90 | | 0.5140 | 97.2189 |
| 2 | 1091 | 505 | 1097 | 7.9 | 8.7014 | 29.1149 |
| 3 | 1257 | 1097 | 1468 | 92.1 | 100.7645 | 252.2252 |

| FIG. 10 | | | | | | |
|---|---|---|---|---|---|---|
| 1 | 15 (LM) | 0 | 72 | | 0.5431 | 96.8544 |
| 2 | 2013 | 1864 | 2026 | 1.9 | 1.0126 | 1.6075 |
| 3 | 2260 | 2026 | 2864 | 98.1 | 52.0452 | 71.9007 |

| FIG. 11 | | | | | | |
|---|---|---|---|---|---|---|
| 1 | 15 (LM) | 0 | 69 | | 0.5194 | 28.2489 |
| 2 | 1428 | 463 | 1434 | 5.4 | 7.9329 | 20.9317 |
| 3 | 1564 | 1434 | 1826 | 94.6 | 138.8152 | 277.9095 |

In conclusion, the present disclosure provides a universal plasmid vector with high stability, which has universal applicability and can solve the problem of plasmid instability caused by polyA sequences in the field of mRNA.

## Claims

1. A nucleic acid molecule, comprising: 1) a transcribable nucleic acid fragment and/or a nucleic acid for introducing a transcribable nucleic acid fragment; 2) a nucleic acid fragment comprising polydeoxyadenosine polyA; and 3) a nucleic acid fragment comprising a gyrase binding site.

2. The nucleic acid molecule according to claim 1, wherein the transcribable nucleic acid fragment encodes a peptide or a protein.

3. The nucleic acid molecule according to claim 1 or 2, wherein the nucleic acid fragment for introducing the transcribable nucleic acid fragment comprises a multiple cloning site or a homologous recombination site.

4. The nucleic acid molecule according to any one of claims 1-3, wherein the polyA comprises at least 50 contiguous deoxyadenosines.

5. The nucleic acid molecule according to any one of claims 1-4, wherein the nucleic acid fragment comprising the polyA comprises at least two elements having a length of at least 20 contiguous deoxyadenosine sequences, and the two elements are spaced apart by at least one non-T deoxynucleotide.

6. The nucleic acid molecule according to any one of claims 1-5, wherein the nucleic acid fragment comprising the gyrase binding site is derived from a virus or a bacterium; preferably, the nucleic acid fragment comprising the gyrase binding site is derived from a virus; more preferably, the nucleic acid fragment comprising the gyrase binding site is derived from a phage.

7. The nucleic acid molecule according to any one of claims 1-5, wherein the nucleic acid fragment comprising the gyrase binding site is derived from a plasmid; preferably, the nucleic acid fragment comprising the gyrase binding site is derived from a ColE1 plasmid.

8. A plasmid vector, comprising: 1) a first nucleic acid fragment encoding an mRNA molecule; 2) a second nucleic acid fragment comprising a polyA; and 3) a third nucleic acid fragment comprising a gyrase binding site.

9. A method for preparing a plasmid vector, comprising transforming the plasmid vector according to claim 8 into *E*. *coli* competent cells, spreading a proper amount of the transformed cells onto a resistant LB culture medium plate for culturing, picking a monoclonal colony and performing sequencing to obtain a positive bacterial solution verified to be correct by sequencing, and then conducting large-scale culturing.

10. A method for amplifying a nucleic acid molecule, comprising propagating the nucleic acid molecule according to claims 1-7 in a host cell and propagating the host cell, wherein the host cell comprises a bacterium or a fungus; the host cell is preferably *E*. *coli*.

11. A method for obtaining an RNA, comprising: amplifying a nucleic acid molecule by the method according to claim 10, and transcribing the nucleic acid molecule *in vitro* as a template into the RNA.

12. A method for obtaining a peptide or a protein, comprising: obtaining an mRNA encoding the peptide or the protein by the method according to claim 11, and translating the mRNA into an amino acid fragment of the peptide or the protein.

13. A system for producing a eukaryotic translatable mRNA, comprising: 1) a plasmid vector comprising the nucleic acid molecule according to claims 1-7; and 2) a host cell.

14. Use of a nucleic acid fragment encoding a gyrase binding site in constructing a plasmid vector, wherein the plasmid vector is used for *in vitro* transcription into RNA.

15. A composition, comprising an RNA, a peptide, or a protein obtained by the method according to claim 11 or 12.

16. The composition according to claim 15, comprising one or more of a pharmaceutically acceptable excipient, a carrier, a buffer, a protectant, a stabilizer, a surfactant, an osmotic pressure regulator, an adjuvant, a preservative, and an inactivator.

17. Use of the nucleic acid molecule according to any one of claims 1-7, the plasmid vector according to claim 8, or the composition according to any one of claims 15-16 in preparing a medicament or a vaccine for treating or preventing a disease.

18. The use according to claim 17, wherein the disease is caused by one or more of the following viruses, bacteria, or protozoa: novel coronavirus, dengue virus, malaria parasite, herpes zoster virus, feline parvovirus, feline calicivirus, feline herpes virus, feline leukemia virus, feline coronavirus, porcine reproductive and respiratory syndrome virus, herpes simplex virus 1, herpes simplex virus 2, encephalitis virus, papillomavirus, varicella zoster virus, Epstein-Barr virus, human cytomegalovirus, human herpes virus 8, human papillomavirus, BK virus, JC virus, smallpox, poliovirus, hepatitis B virus, human bocavirus, parvovirus B19, human astrovirus, Norwalk virus, coxsackievirus, hepatitis A virus, rhinovirus, severe acute respiratory syndrome virus, hepatitis C virus, yellow fever virus, West Nile virus, rubella virus, hepatitis E virus, human immunodeficiency virus (HIV), influenza virus A or B, Guanarito virus, Junin virus, Lassa virus, Machupo virus, Sabia virus, Crimean-Congo hemorrhagic fever virus, Ebola virus, Marburg virus, measles virus, mumps virus, parainfluenza virus, respiratory syncytial virus, human metapneumovirus, Hendra virus, Nipah virus, rabies virus, hepatitis D, rotavirus, orbivirus, *E*. *coli* virus, human enterovirus, Japanese encephalitis virus, vesicular herpes virus, eastern equine encephalitis virus, Hantaan virus, Middle East respiratory syndrome coronavirus, chikungunya virus, or Banna virus.
